# DEMANDE DE BREVET EUROPEEN

(11) **EP 1 829 523 A1**
(43) Date de publication de la demande: **05.09.2007**
(21) Numéro de dépôt: 07300824.5
(22) Date de dépôt: 26.02.2007
(51) Int. Cl.: A61K 8/49, A61Q 7/00, A61K 31/52

(54) **Utilisation cosmétique d'un dérivé N-oxyde de théophylline**

(30) Priorité: 01.03.2006 FR 0650714
(71) Demandeur: L'ORÉAL, 75008 Paris (FR)
(72) Inventeur: Dalko, Maria, 91190 Gif-sur-Yvette (FR)
(74) Mandataire: Le Coupanec, Pascale A.M.P.

(57) **Abrégé**

La présente invention concerne l'utilisation cosmétique d'au moins un dérivé N-oxyde de la formule (I) 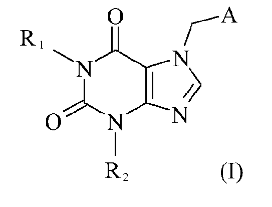 A comportant un groupe nitrate comme agent précurseur de NO.

## Description

La présente invention se rapporte à l'utilisation d'au moins un dérivé N-oxyde de la théophylline ainsi que ses sels cosmétiquement acceptables, dans une composition cosmétique destinée à induire et/ou stimuler la croissance des fibres kératiniques humaines et en particulier les cheveux et les cils et/ou freiner leur chute.

L'invention a aussi pour objet un procédé de traitement cosmétique destiné à stimuler la croissance des fibres kératiniques humaines telles que les cheveux et les cils et/ou freiner leur chute.

L'invention concerne également de nouveaux dérivés de N-oxyde théophylline, présentant une activité précurseur de NO. L'invention se rapporte en outre à des compositions cosmétiques ou dermatologiques comprenant ces nouveaux dérivés N-oxyde de la théophylline.

La croissance des cheveux et leur renouvellement sont principalement déterminés par l'activité des follicules pileux et de leur environnement matriciel. Leur activité est cyclique et comporte essentiellement 3 phases à savoir la phase anagène, la phase catagène et la phase télogène.

A la phase anagène (phase active ou phase de croissance) qui dure plusieurs années et au cours de laquelle les cheveux s'allongent, succède une phase catagène très courte et transitoire qui dure quelques semaines. Au cours de cette phase, le cheveu subit une évolution, le follicule s'atrophie et son implantation dermique apparaît de plus en plus haute.

La phase terminale ou phase télogène, qui dure quelques mois, correspond à une phase de repos du follicule et le cheveu finit par tomber.

A la fin de cette période de repos, les cheveux tombent et un autre cycle recommence. La chevelure se renouvelle donc en permanence et sur les 150 000 cheveux environ que comporte une chevelure, à chaque instant, 10 % environ sont au repos et seront remplacés en quelques mois.

A l'âge adulte, le système vasculaire de la peau est parachevé et ne se modifie plus sauf au niveau des follicules pileux où il subit d'importantes variations à chaque cycle pilaire.

Les follicules pileux sont en effet une structure cutanée richement innervée et très vascularisée. De façon générale, le phénomène de développement de la microcirculation est appelé angiogénèse. Au début de chaque phase anagène, il est nécessaire de développer une forte activation de l'angiogénèse afin de re-développer le micro-réseau vasculaire périfolliculaire. L'involution de ce réseau et la disparition des capillaires de la papille dermique vont de pair avec le changement de phase et le passage en phase catagène. A ce stade, les micro-capillaires sanguins collapsent et disparaissent.

Parallèlement, dans les zones alopéciques, une fibrose périfolliculaire s'installe, les follicules se miniaturisent cycle après cycle et progressivement, la vascularisation des bulbes s'ammoindrie.

Le phénomène d'angiogénèse observé au cours de la phase anagène dépend de nombreux facteurs trophiques, de cytokines ou d'autres molécules biologiquement actives apportées par la circulation sanguine ou localement produites en particulier par les fibroblastes de la papille dermique ou les kératinocytes du bulbe capillaire. Parmi ces facteurs trophiques on peut citer le facteur de croissance des cellules endothéliales (appelé également vascular endothelial growth factor (VEGF) en terminologie anglo-saxonne). Ce facteur est essentiel pour l'angiogénèse et augmente la perméabilité vasculaire. Des études ont montré que l'expression de ce facteur était augmentée au cours de la phase anagène du cycle pilaire. Ainsi, ce facteur contribue au maintien d'une microvascularisation fonctionnelle autour du follicule pileux et notamment de la base du bulbe et de la papille dermique, ainsi qu'à l'apport de nutriments nécessaire à la bonne croissance du cheveu.

La microcirculation périfolliculaire joue donc un rôle primordial dans le processus de croissance pilaire en apportant les facteurs et les nutriments nécessaires à la croissance de ce follicule.

La chute ou perte naturelle des cheveux peut être estimée, en moyenne, à quelques cent cheveux par jour pour un état physiologique normal. Ce processus de renouvellement physique permanent subit une évolution naturelle au cours du vieillissement, les cheveux deviennent plus fins et leurs cycles plus courts.

Par ailleurs, dans certaines dermatoses du cuir chevelu à caractéristique inflammatoire, telles que par exemple le psoriasis ou les dermatites séborrhéiques, la chute des cheveux peut être fortement accentuée et les cycles de renouvellement des follicules peuvent être fortement perturbés.

En outre, différentes causes peuvent entraîner une perte importante, temporaire ou définitive, des cheveux. Il peut s'agir de chute et d'altération des cheveux au décours d'une grossesse (*post partum*), au cours d'états de dénutrition ou de déséquilibres alimentaires ou encore au cours d'états d'asthénie ou de dysfonctionnement hormonal comme cela peut être le cas au cours ou au décours de la ménopause. Il peut également s'agir de chute ou d'altérations des cheveux en relation avec des phénomènes saisonniers. Il peut également s'agir d'une alopécie qui est essentiellement due à des perturbations du renouvellement capillaire qui entraîne dans un premier temps l'accélération de la fréquence des cycles au détriment de la qualité des cheveux puis de la quantité. Il se produit un appauvrissement progressif de la chevelure et une miniaturisation progressive de ceux-ci, conjointement à un isolement des bulbes par une progression de l'épaisseur de la matrice collagénique perifolliculaire et de la gaine conjonctive externe. La revascularisation est donc rendue plus difficile cycle après cycle. Les cycles de croissance successifs aboutissent à des cheveux de plus en plus fins et de plus en plus courts, se transformant peu à peu en un duvet non pigmenté. Des zones sont touchées préférentiellement, notamment les golfes temporaux ou frontaux chez l'homme et, chez la femme, on constate une alopécie diffuse du vertex.

De par le rôle primordial de la microcirculation périfolliculaire énoncé plus haut, tout défaut de cette dernière entraînera une diminution de l'apport des éléments nutritifs et gazeux (Oxygène notamment) nécessaires à la croissance pilaire conduisant à des troubles de la croissance du cheveu et la mise en place progressive d'une alopécie.

Le terme alopécie recouvre aussi toute une famille d'atteintes du follicule pileux ayant pour conséquence finale la perte définitive, partielle ou générale des cheveux. Il s'agit plus particulièrement de l'alopécie androgénique. Dans un nombre important de cas, la chute précoce des cheveux survient chez des sujets prédisposés génétiquement, il s'agit alors d'alopécie andro-chrono-génétique ; cette forme d'alopécie concerne notamment les hommes.

Il est connu, par ailleurs, que certains facteurs tels qu'un déséquilibre hormonal, un stress physiologique ou la malnutrition peuvent accentuer le phénomène de chute des cheveux.

De façon générale, tout facteur entraînant une augmentation de l'apport sanguin au niveau du follicule soit en activant l'angiogénèse soit en s'opposant à sa régression soit en agissant sur les microvaisseaux pour limiter leur constriction, aura un effet bénéfique sur l'apport énergétique nécessaire à la bonne croissance de ce même follicule.

L'une des voies explorées jusqu'à ce jour pour trouver des méthodes pour lutter contre la chute des cheveux est le maintien de la vascularisation autour du follicule. Différentes substances actives ont été développées en ce sens, mais cependant ces substances présentent de nombreux effets indésirables. En particulier, elles présentent des activités multiples, pouvant perturber l'équilibre ionique et physiologique des cellules cutanées. Autrement dit, leur activité multiple rend difficile leur contrôle d'action sur les cellules.

Un de ces composés est le vérapamil, qui est un antagoniste puissant (IC₅₀ = 38nM) des canaux calciques de type L. Le vérapamil et d'autres antagonistes calciques comme le diltiazem et la nifédipine sont décrits comme étant actifs dans le traitement de la chute des cheveux, en particulier par leurs effets sur la microcirculation (brevet Shiseido : 88JP062680 et brevet Coppe J : 89BE-000305).

Il existe en outre des brevets dérivants l'utilisation des donneurs de NO (monoxyde d'azote) pour application topique, pour stimuler la croissance de cheveux en agissant sur la microcirculation du cuir chevelu. Ainsi, la demande de brevet EP 327 263 décrit l'utilisation des composés producteurs du radical NO en association avec des agents réducteurs, des anti-oxydants et avec des piégeurs de radical hydroxyle. La demande de brevet WO 99/13717 décrit seulement l'utilisation de l'arginine et ses dérivés, comme substrat de NO-synthase, pour la formation *in vivo* de NO et leur utilisation (entre autres) dans le traitement de l'alopécie. Une demande de brevet JP 07316023 décrit également l'utilisation de l'arginine et ses dérivés dans le traitement de l'alopécie.

La présente invention se rapporte à l'utilisation de composés particuliers permettant de remédier aux inconvénients énoncés ci-dessus. Ces composés présentent en outre, une activité locale, spécifique. Ils assurent une revascularisation du follicule pileux, de sa région périphérique et/ou du cheveu après chaque cycle de croissance, ainsi qu'une inhibition de la formation et de l'installation de la fibrose périfolliculaire, facteur aggravant une mauvaise vascularisation du cheveu.

Dans le document DE 924085 certains dérivés d'alkylnitrate de théophilline sont décrits comme vasodepresseurs. Aucune application cosmétique n'est par ailleurs décrite dans ce document. Les trois composés divulgués ont les formules suivantes :

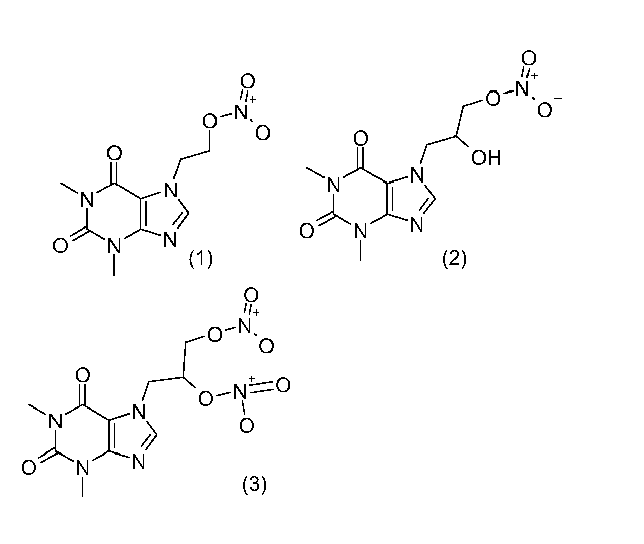

Dans le document Eckstein et al., « Dissertationes Pharmaceuticae » (1964) 16(1), 43-50, la préparation de dérivés nitrate à partir de dérivés hydroxyalkylthéophyllines est décrite. Ces composés sont décrits comme étant des médicaments de type dérivés xanthiques (également appelées bases xanthiques). Les deux composés synthétisés ont les formules qui suivent :

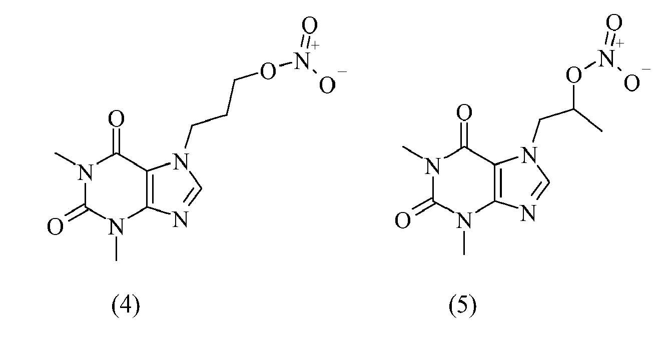

D'une façon surprenante, les inventeurs ont démontré que les dérivés N-oxyde de théophylline sont stables en solution, à savoir ne donnent pas de libération spontanée de NO, tout en donnant lieu à une libération de NO en présence des kératinocytes. Ces composés peuvent donc être considérés comme des précurseurs de NO et se trouvent donc particulièrement adaptés à une application topique, notamment dans le but de stimuler la croissance des cheveux et/ou freiner leur chute et/ou augmenter leur densité.

Les inventeurs ont, en outre, trouvé que ces dérivés pouvaient avoir un effet bénéfique sur la pousse des cils mais aussi de certains poils humains. Ces dérivés ont, en outre, un effet bénéfique sur la peau, les lèvres et les ongles.

Selon un de ses aspects, l'invention a donc pour objet l'utilisation cosmétique d'un dérivé N-oxyde de théophylline ou l'un de ses sels de formule (1) 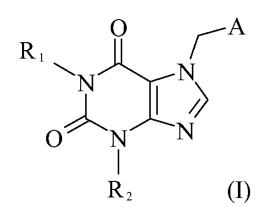 où A peut prendre l'une des deux formules (II) ou (III) suivantes : 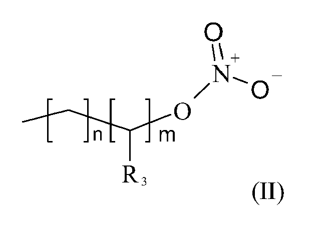 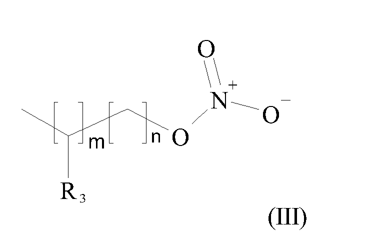 et dans laquelle :
- n représente un entier allant de 0 à 5,
- m représente un entier allant de 0 à 1, à condition que m et n ne soient pas égaux à 0 simultanément,
- R₁ et R₂ représentent indépendamment un radical (C₁-C₅)alkyle, linéaire, ramifié ou cyclique, saturé ou insaturé et,
- R₃ représente un groupe (C₁-C₄)alkyle, un groupe hydroxy, un groupe (C₁-C₄)alkoxy ou un groupe nitrate,
comme agent précurseur de NO.

Par « augmenter la densité des fibres kératiniques », et notamment la densité capillaire, on entend augmenter le nombre de fibres kératiniques, notamment de cheveux, par cm² de peau telle que le cuir chevelu.

Selon un autre de ses aspects, l'invention a pour objet des composés de formule (I) et leurs sels, à l'exclusion du composé de formule (1) non salifié pour lequel A prend la formule (III), n est égal à 1, m est égal à 0 et R₁ et R₂ représentent chacun un groupe méthyle ; du composé de formule (2) non salifié pour lequel A prend la formule (III), n et m sont égaux à 1, R₁ et R₂ représentent chacun un groupe méthyle et R₃ représente un groupe hydroxyle ; du composé de formule (3) non salifié pour lequel A prend la formule (III), n et m sont égaux à 1, R₁ et R₂ représentent chacun un groupe méthyle et R₃ représente un groupe nitrate ; du composé de formule (4) non salifié pour lequel A prend la formule (III), n est égal à 2, m est égal à 0 et R₁ et R₂ représentent chacun un groupe méthyle et du composé de formule (5) non salifié pour lequel A prend la formule (III), n est égal à 0, m est égal à 1, R₁ et R₂ représentent chacun un groupe méthyle et R₃ représente un groupe méthyle.

Selon un autre de ses aspects, l'invention a pour objet une composition cosmétique contenant dans un milieu physiologiquement acceptable une quantité efficace d'au moins un composé de formule (I) ou l'un de ses sels.

Selon encore un autre de ses aspects, l'invention a également pour objet une composition dermatologique contenant dans un milieu physiologiquement acceptable une quantité efficace d'au moins un composé de formule (I) ou l'un de ses sels tels que défini précédemment à l'exclusion du composé de formule (1) non salifié pour lequel A prend la formule (III), n est égal à 1, m est égal à 0 et R₁ et R₂ représentent chacun un groupe méthyle ; du composé de formule (2) non salifié pour lequel A prend la forme (III), n et m sont égaux à 1, R₁ et R₂ représentent chacun un groupe méthyle et R₃ représente un groupe hydroxyle ; du composé de formule (3) non salifié pour lequel A prend la formule (III), n et m sont égaux à 1, R₁ et R₂ représentent chacun un groupe méthyle et R₃ représente un groupe nitrate ; du composé de formule (4) non salifié pour lequel A prend la formule (III), n est égal à 2, m est égal à 0 et R₁ et R₂ représentent chacun un groupe méthyle et du composé de formule (5) non salifié pour lequel A prend la formule (III), n est égal à 0, m est égal à 1, R₁ et R₂ représentent chacun un groupe méthyle et R₃ représente un groupe méthyle. Cette composition dermatologique est en particulier destinée à induire et/ou stimuler la croissance des fibres kératiniques et/ou freiner leur chute et/ou augmenter leur densité.

Selon encore un autre de ses aspects, l'invention a également pour objet une composition dermatologique topique contenant dans un milieu physiologiquement acceptable une quantité efficace d'au moins un composé de formule (I) ou l'un de ses sels, tels que défini précédemment

Les « fibres kératiniques » humaines auxquelles s'applique l'invention sont notamment les cheveux, les sourcils, les cils, les poils de barbe, de moustache et les poils pubiens. Plus spécialement, l'invention s'applique aux cheveux et/ou aux cils humains.

En particulier, l'invention se rapporte à l'utilisation cosmétique d'au moins un composé de formule (I) ou de l'un de ses sels dans une composition cosmétique de soin capillaire d'être humain, comme agent pour traiter l'alopécie d'origine naturelle et en particulier l'alopécie androgénique ou andro-chrono-génétique, ou à l'utilisation d'au moins un composé de formule (I) ou de l'un de ses sels, pour la préparation d'une composition capillaire d'être humain, destinée à traiter l'alopécie d'origine naturelle et en particulier androgénique ou andro-chrono-génétique. Ainsi, cette composition permet de maintenir en bon état la chevelure et/ou lutter contre la chute naturelle des cheveux en particulier des hommes.

L'invention a encore pour objet l'utilisation cosmétique d'au moins un dérivé de formule (I) ou de l'un de ses sels, tel que défini ci-dessus, dans une composition cosmétique de soin et/ou de maquillage des cils d'êtres humains ou pour la préparation d'une composition de soin et/ou de traitement des cils d'êtres humains, pour ou destinée à induire et/ou stimuler la croissance des cils et/ou augmenter leur densité. Cette composition permet ainsi de maintenir en bon état les cils et/ou améliorer leur état et/ou leur aspect.

Selon encore un autre de ses aspects, l'invention a encore pour objet l'utilisation d'au moins un composé de formule (I) ou l'un de ses sels, pour la préparation d'une composition de soin et/ou de traitement des fibres kératiniques d'êtres humains, déstinée à induire et/ou stimuler la croissance des fibres kératiniques et/ou freiner leur chute et/ou augmenter leur densité.

Elle a encore pour objet l'utilisation d'au moins un composé de formule (I) ou de l'un de ses sels, pour la fabrication d'une composition destinée à traiter les désordres liés à une réduction de la microcirculation ou vascularisation cutanée et notamment des follicules pileux chez l'être humain.

Comme désordres liés à une réduction de la microcirculation, on peut citer le dépôt de collagène, le psoriasis, les ulcères chroniques, l'accroissement de cellulite et/ou de graisse, le syndrome de Raynaud et la modulation de la pigmentation de la peau et du cheveu. Ainsi, les dérivés de formule (I) peuvent être utilisés pour la préparation de compositions anti-âge, amincissante ou conférant à la peau un éclat du teint ou une luminosité (appelé effet bonne mine) ou aux lèvres de la couleur ou la préparation d'une composition destinée à lutter contre le syndrome de Raynaud, les ulcères ou le psoriasis. Cette composition permet notamment un lissage et/ou une diminution des rides.

Aussi, l'invention a pour objet l'utilisation cosmétique d'au moins un dérivé de formule (I) ou l'un de ses sels, dans une composition cosmétique anti-âge, anti-ride, amincissante ou conférant à la peau un éclat du teint ou une luminosité du teint ou aux lèvres de la couleur, et l'utilisation d'au moins un composé de formule (I) ou l'un de ses sels, pour la préparation d'une composition physiologiquement acceptable, destinée à traiter les signes du vieillissement intrinsèques et/ou extrinsèques, les surcharges pondérales, les ulcères, le syndrome de Raynaud, le psoriasis et/ou améliorer la cicatrisation cutanée. Les signes du vieillissement extrinsèques sont en particulier ceux dus aux rayonnements ultraviolets (lampes U.V. ou soleil intense).

Par leur activité précurseur de NO, les dérivés selon l'invention permettent aussi de lutter contre les mycoses cutanées, les désordres inflammatoires comme les érythèmes notamment solaires ou de lupus, les réactions d'hypersensibilité de contact et/ou les manifestations allergiques, l'eczéma, les prurits, les peaux sensibles, y compris le cuir chevelu sensible, mais aussi contre les hypermélanoses.

Aussi, l'invention a encore pour objet l'utilisation d'au moins un composé de formule (I) ou l'un de ses sels, pour la fabrication d'une composition physiologiquement acceptable, destinée à traiter les mycoses cutanées, les désordres inflammatoires, les réactions d'hypersensibilité de contact, les manifestations allergiques, l'eczéma, les prurits, les peaux sensibles et/ou l'hypermélanose.

La présente invention a également pour objet un procédé de traitement cosmétique des fibres kératiniques humaines et/ou de la peau y compris le cuir chevelu, destiné notamment à stimuler la croissance des fibres kératiniques humaines telles que les cheveux et les cils d'être humain et/ou freiner leur chute, caractérisé par le fait qu'il consiste à appliquer sur les fibres kératiniques humaines et/ou la peau, une composition cosmétique comprenant une quantité efficace d'au moins un composé de formule (I) ou d'un de ses sels, à laisser celle-ci en contact avec les fibres kératiniques et/ou la peau, et éventuellement à rincer les fibres kératiniques et/ou la peau.

Ce procédé de traitement présente les caractéristiques d'un procédé cosmétique dans la mesure où il permet d'améliorer l'esthétique des fibres kératiniques et en particulier des cheveux et des cils en leur donnant une plus grande vigueur et un aspect amélioré. En outre, il peut être utilisé quotidiennement pendant plusieurs mois, sans prescription médicale.

Plus spécialement, la présente invention a pour objet un procédé de soin cosmétique des cheveux et/ou du cuir chevelu humains, en vue d'améliorer leur état et/ou leur aspect, caractérisé en ce qu'il consiste à appliquer sur les cheveux et/ou le cuir chevelu, une composition cosmétique comprenant au moins un dérivé de formule (I) ou l'un de ses sels, à laisser celle-ci au contact des cheveux et/ou du cuir chevelu, et éventuellement à rincer les cheveux et/ou le cuir chevelu.

L'invention a encore pour objet un procédé de soin cosmétique et/ou de maquillage ces cils humains, en vue d'améliorer leur état et/ou leur aspect, caractérisé en ce qu'il consiste à appliquer une composition de mascara comprenant au moins un composé de formule (I) ou l'une de ses sels et à laisser celle-ci au contact des cils. Cette composition de mascara peut être appliquée seule ou en sous-couche d'un mascara pigmenté classique et être éliminée comme un mascara pigmenté classique.

### Utilisation cosmétique des composés

Avantageusement, les composés de formule (I), sous forme salifiée ou non, présentent une activité, exercée au niveau de la peau et en particulier du cuir chevelu. Sans que cela ne constitue une quelconque limitation à l'invention, les inventeurs ont émis l'hypothèse que les composés présentent un mécanisme d'action du type mécanisme de libération du NO qui peut s'expliquer comme suit. Les composés de formule (I) ont la particularité d'être inactifs, en l'absence d'enzyme et en particulier d'hydrolases.

Autrement dit, les composés de formule (I) inactifs, libèrent du NO *in vivo*, sous l'action des enzymes cutanées (par exemple les hydrolases), qui se traduit par un effet bénéfique sur la microcirculation : le NO libéré ayant un effet vasodilatateur.

Le schéma réactionnel, par exemple dans le cas où m est égal à 0, peut être le suivant : 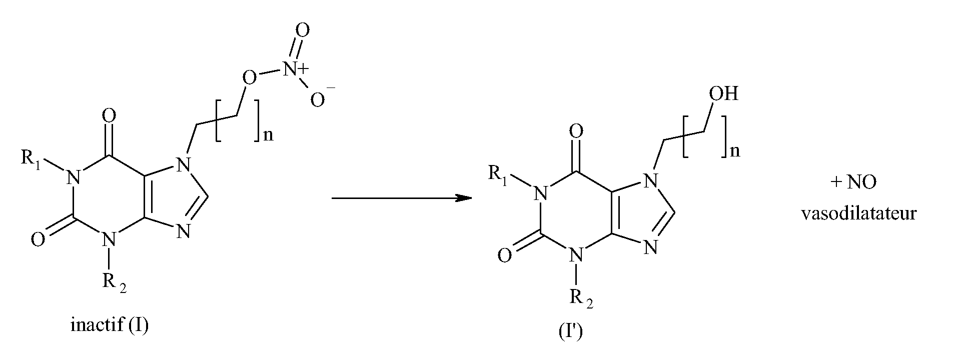 avec n, R₁ et R₂ ayant la signification précédemment indiquée.

Dans la suite du texte, et sauf mention exprès, l'emploi du terme composé de formule (I) doit être compris comme signifiant aussi bien le composé de formule (I) sous forme acide ou basique, que l'un de ses sels.

Ils peuvent aussi être sous forme tautomère. Ces composés peuvent être des énantiomères et/ou des diastéréoisomères ou un mélange de ces isomères, en particulier un mélange racémique.

Comme exemple de radical alkyle utilisable dans l'invention, on peut citer les radicaux méthyle, éthyle, propyl, isopropyle, n-butyle, isobutyl, terbutyle, n-pentyle.

Selon l'invention, les composés de formule (I) sont sous forme isolée, c'est-à-dire non polymérique.

Par sels de composé de formule (I), on entend selon l'invention, les sels organiques ou inorganiques d'un composé de formule (I).

Comme sels inorganiques utilisables selon l'invention on peut citer : les sels de sodium ou de potassium ainsi que les sels de zinc (Zn²⁺), de calcium (Ca²⁺), de cuivre (Cu²⁺), de fer (Fe²⁺), de strontium (Sr²⁺), de magnésium (Mg²⁺) et de manganèse (Mn²⁺) ; les hydroxydes, les carbonates, les chlorures, les sulfates, les citrates, les acétates, les lactates.

Les sels organiques utilisables selon l'invention sont par exemple les sels de tri-éthanolamine, mono-éthanolamine, di-éthanolamine, hexadécylamine et N,N,N',N'-tétrakis-(hydroxy-propyl-2) éthylène diamine, tris-hydroxyméthylaminométhane.

Dans le cadre de la présente invention, on préfère l'utilisation cosmétique des composés de l'invention, pour lesquels R₁ et R₂ représentent indépendamment un groupe (C₁-C₅)alkyle non substitué.

L'utilisation cosmétique des composés de l'invention pour lesquels R₁ et R₂ sont chacun un groupe méthyle est également avantageuse.

Parmi ces deux catégories de composés, on préfère par ailleurs l'utilisation cosmétique des composés de formule (I) selon l'invention pour lesquels m est égal à 0, n est un entier allant de 1 à 3, et plus particulièrement pour lesquels n est égal à 1.

A la connaissance du demandeur, aucun document de l'art antérieur ne décrit ni ne suggère que les composés de formule (I) ou leurs sels aient la propriété d'induire et/ou de stimuler la croissance des fibres kératiniques et/ou de freiner leur chute ni que ces composés peuvent être utilisés par voie topique pour augmenter la densité de ces fibres.

### Nouveaux composés

Parmi les composés de formule (I) et leurs sels, certains composés sont nouveaux et font partie de l'invention. Il s'agit de composés de formule (I) et leurs sels à l'exclusion du composé de formule (1) non salifié pour lequel A prend la formule (III), n est égal à 1, m est égal à 0 et R₁ et R₂ représentent chacun un groupe méthyle ; du composé de formule (2) non salifié pour lequel A prend la formule (III), n et m sont égaux à 1, R₁ et R₂ représentent chacun un groupe méthyle et R₃ représente un groupe hydroxyle ; du composé de formule (3) non salifié pour lequel A prend la formule (III), n et m sont égaux à 1, R₁ et R₂ représentent chacun un groupe méthyle et R₃ représente un groupe nitrate ; du composé de formule (4) non salifié pour lequel A prend la formule (III), n est égal à 2, m est égal à 0 et R₁ et R₂ représentent chacun un groupe méthyle et du composé de formule (5) non salifié pour lequel A prend la formule (III), n est égal à 0, m est égal à 1, R₁ et R₂ représentent chacun un groupe méthyle et R₃ représente un groupe méthyle.

Ces composés sont en effet décrits dans le document DE 924 065 et dans le document « Dissertationes Pharmaceuticae » (1964) 16(1), 43-50, comme cela a déjà été mentionné plus haut.

Le tableau ci-après rassemble certains composés nouveaux qui trouvent notamment leurs applications dans les utilisations déjà décrites ci-dessus, dans le cadre de la présente invention. Lorsque la chaîne latérale portant le groupe nitrate est ramifiée, A prend la formule (III).

**Tableau 1**

| **N°** | **Nom chimique** | **Formule** | | | | |
|---|---|---|---|---|---|---|
| | | **R₁** | **R₂** | **n** | **m** | **R₃** |
| 1 | 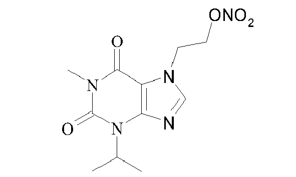 Nitrate de 1-méthyl-3-isopropyle-7-(2-hydroxy éthyl)- xanthine | méthyle | isopropyle | 1 | 0 | - |
| 2 | 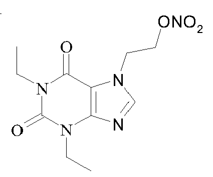 Nitrate de 1,3-diéthyl-7-(2-hydroxy éthyl)-xanthine | éthyle | éthyle | 1 | 0 | - |
| 3 | 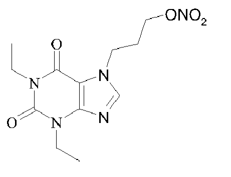 Nitrate de 1,3-diéthyl-7-(3-hydroxypropyl)-xanthine | éthyle | éthyle | 2 | 0 | - |
| 4 | 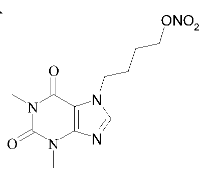 Nitrate de 1,3-diméthyl-7-(4-hydroxbutyle)-xanthine | méthyle | méthyle | 3 | 0 | - |
| 5 | 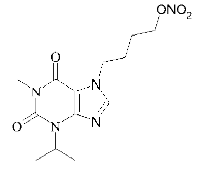 Nitrate de 1-méthyl-3-isopropyl-7-(4-hydroxybutyle)-xanthine | méthyle | isopropyle | 3 | 0 | - |
| 6 | 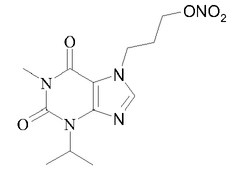 Nitrate de 1-méthyl-3-isopropyl-7-(3-hydroxypropyle)-xanthine | méthyle | isopropyle | 2 | 0 | - |
| 7 | 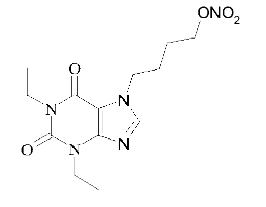 Nitrate de 1,3-diéthyl-7-(4-hydroxybutyle)-xanthine | éthyle | éthyle | 3 | 0 | - |
| 8 | 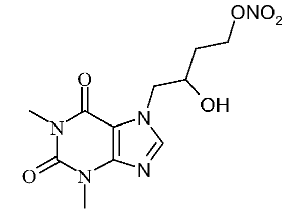 4'-Nitrate de 1,3-diméthyl-7-(2,4-dihydroxy butyle)-xanthine | méthyle | méthyle | 2 | 1 | hydroxy |
| 9 | 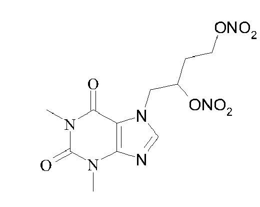 2',4'-dinitrate de 1,3-diméthyl-7-(2,4,-dihydroxybutyle)-xanthine | méthyle | méthyle | 2 | 1 | nitrate |
| 10 | 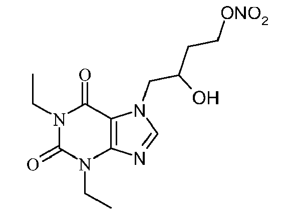 4'-nitrate de 1,3-diéthyl-7-(2,4-dihydroxybutyle)-xanthine | éthyle | éthyle | 2 | 1 | hydroxy |
| 11 | 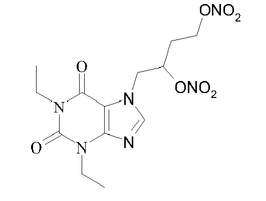 2',4'-dinitrate de 1,3-diéthyl-7-(2,4-dihydroxybutyle)-xanthine | éthyle | éthyle | 2 | 1 | nitrate |
| 12 | 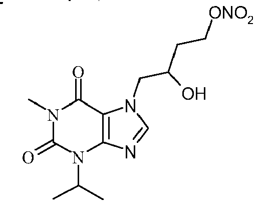 4'-nitrate de 1-méthyl-3-isopropyl-7 (2,4-dihydroxbutyle) xanthine | méthyle | isopropyle | 2 | 1 | hydroxy |
| 13 | 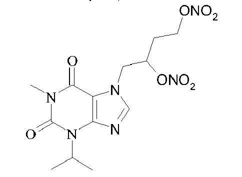 2',4'-dinitrate de 1-méthyl-3-isopropyl-7-(2,4-dihydroxbutyle) xanthine | méthyle | isopropyle | 2 | 1 | nitrate |

### Procédé

Les composés de formule (I) salifiés ou non peuvent être synthétisés selon la méthode décrite dans « Dissertationes Pharmaceuticae » (1964) 16(1), 43-50.

Les produits de départ pour les composés de formule (I), à savoir les hydroxyalkyl-théophylline, sont commerciaux ou peuvent être obtenus selon le procédé décrit dans la demande de brevet CH 314 636 à partir des produits de départ correspondants par les voies de synthèse présentées ci-dessous dans le schéma 1 :

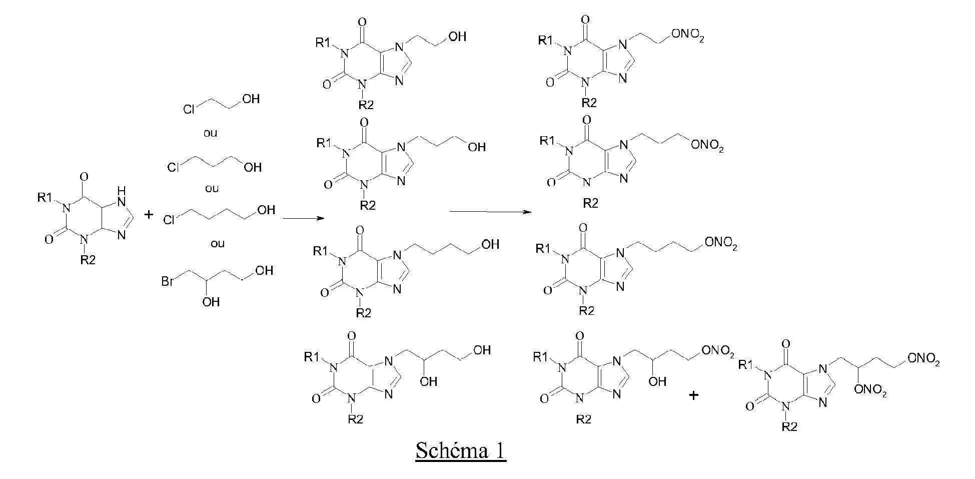

### Composition

« Au moins un », selon l'invention, signifie un ou plusieurs (2, 3 ou plus). En particulier, la composition peut contenir un ou plusieurs composés de formule (I).

La quantité efficace d'un composé de formule (I) ou de l'un de ses sels correspond à la quantité nécessaire pour obtenir le résultat désiré (en particulier à savoir augmenter la densité des fibres kératiniques ou favoriser leur pousse). L'homme du métier est donc en mesure d'évaluer cette quantité efficace qui dépend de la nature du composé utilisé, de la personne à laquelle on l'applique, et du temps de cette application.

Dans la suite du texte, et sauf indication contraire, les quantités des différents ingrédients de la composition sont données en pourcentage en poids par rapport au poids total de la composition.

Pour donner un ordre de grandeur, selon l'invention, le ou les composé(s) de formule (I) ou de l'un de ses sels peu(ven)t être présent(s) en un teneur allant de 10⁻³ % à 5 % du poids total de la composition et préférentiellement en une quantité représentant de 10⁻² % à 2 % du poids total de la composition, et de façon encore plus préférée de 0,1 % à 2%.

La composition doit contenir un milieu physiologiquement acceptable non toxique et susceptible d'être appliqué sur la peau, y compris le cuir chevelu et les paupières, les phanères tels que les fibres kératiniques ou les lèvres d'êtres humains. Par « cosmétique », on entend au sens de l'invention une composition d'aspect, d'odeur et de toucher agréables.

Le composé de formule (I), peut être utilisé dans une composition qui doit être ingérée, injectée ou appliquée sur la peau ou les fibres kératiniques (sur toute zone cutanée ou fibres à traiter).

Selon l'invention, le composé de formule (I) peut être utilisé par la voie orale en une quantité de 0,1 à 300 mg par jour, 5 à 10 mg/j.

Une composition préférée de l'invention est une composition à usage cosmétique et en particulier d'application topique sur la peau et les fibres kératiniques, et plus spécialement sur le cuir chevelu, les cheveux et les cils.

Cette composition peut se présenter sous toutes formes galéniques connues adaptées au mode d'utilisation.

Pour une application topique sur la peau, y compris le cuir chevelu, la composition peut avoir la forme d'une solution ou suspension aqueuse, alcoolique, hydro-alcoolique ou huileuse, d'une émulsion ou dispersion de consistance plus ou moins fluide et notamment liquide ou semi-liquide, obtenue par dispersion d'une phase grasse dans une phase aqueuse (H/E) ou inversement (E/H), d'une dispersion ou émulsion solide (H/E) ou (E/H), d'un gel aqueux, hydro-alcoolique ou huileux plus ou moins fluide ou solide, d'une poudre libre ou compactée à utiliser telle quelle ou à incorporer dans un milieu physiologiquement acceptable, ou encore de microcapsules ou microparticules, de dispersions vésiculaires de type ionique et/ou non ionique.

On peut également envisager une composition sous forme de mousse ou encore sous forme de spray ou d'aérosol comprenant alors un agent propulseur sous pression.

La composition peut ainsi se présenter sous forme d'une lotion, sérum, lait, crème H/E ou E/H, gel, onguent, pommade, poudre, baume, patch, tampon imbibé, savon, pain, mousse.

En particulier la composition à application sur le cuir chevelu ou les cheveux peut se présenter sous forme d'une lotion de soin capillaire, par exemple d'application journalière ou bi-hebdomadaire, d'un shampooing ou d'un après-shampooing capillaire, en particulier d'application bi-hebdomadaire ou hebdomadaire, d'un savon liquide ou solide de nettoyage du cuir chevelu d'application journalière, d'un produit de mise en forme de la coiffure (laque, produit pour mise en pli, gel coiffant), d'un masque traitant, d'une crème ou d'un gel moussant de nettoyage des cheveux. Elle peut encore se présenter sous forme de teinture ou de mascara capillaire à appliquer au pinceau ou au peigne.

Par ailleurs, pour une application sur les cils ou les poils, la composition à laquelle s'applique l'invention peut se présenter sous forme d'un mascara, pigmenté ou non, à appliquer à la brosse sur les cils ou encore sur les poils de barbe ou de moustache.

Pour une composition à usage par injection, la composition peut se présenter sous forme de lotion aqueuse ou de suspension huileuse par exemple sous forme de sérum. Pour un usage par voie orale, la composition peut se présenter sous forme de capsules, de granulés, de sirops buvables ou de comprimés.

Selon un mode de réalisation particulier, la composition selon l'invention se présente sous forme de crème ou lotion capillaire, de shampooing ou d'après-shampooing capillaire, de mascara capillaire ou pour cils.

Les quantités des différents constituants du milieu physiologique de la composition selon l'invention sont ceux généralement utilisées dans les domaines considérés. En outre, ces compositions sont préparées selon les méthodes usuelles.

Lorsque la composition est une émulsion, la proportion de la phase grasse peut aller de 2 % à 80 % en poids, et de préférence de 5 % à 50 % en poids par rapport au poids total de la composition. La phase aqueuse est ajustée en fonction de la teneur en phase grasse et en composé(s) (I) ainsi que de celle des éventuels ingrédients additionnels, pour obtenir 100 % en poids. En pratique la phase aqueuse représente de 5 % à 99,9 % en poids.

La phase grasse peut contenir des composés gras ou huileux, liquides à température ambiante (25 °C) et pression atmosphérique (760 mm de Hg), généralement appelés huiles. Ces huiles peuvent être compatibles ou non entre elles et former une phase grasse liquide macroscopiquement homogène ou un système bi- ou triphasique.

La phase grasse peut, en plus des huiles, contenir des cires, des gommes, des polymères lipophiles, des produits « pâteux » ou visqueux contenant des parties solides et des parties liquides.

La phase aqueuse contient de l'eau et éventuellement un ingrédient miscible en toute proportion à l'eau comme les alcools inférieurs en C₁ à C₈ tel que l'éthanol, l'isopropanol, les polyols comme le propylène glycol, le glycérol, le sorbitol ou encore l'acétone ou l'éther.

Les émulsionnants et co-émulsionnants utilisés pour l'obtention d'une composition sous forme d'émulsion sont ceux généralement utilisés dans les domaines cosmétique et pharmaceutique. Leur nature est, en outre, fonction du sens de l'émulsion. En pratique, l'émulsionnant et éventuellement le co-émulsionnant sont présents, dans la composition, en une proportion allant de 0,1 % à 30 % en poids, de préférence de 0,5 à 20 % en poids et mieux de 1 à 8 %. L'émulsion peut, en outre, contenir des vésicules lipidiques et notamment des liposomes.

Lorsque la composition est sous forme d'une solution ou d'un gel huileux, la phase grasse peut représenter plus de 90 % du poids total de la composition.

Avantageusement, la composition est une solution ou suspension aqueuse, alcoolique ou hydro-alcoolique et mieux une solution ou suspension eau/éthanol. La fraction alcoolique peut représenter de 5 % à 99,9 % et mieux de 8 % à 80 %.

Pour une application mascara, la composition est une dispersion de cire-dans-eau ou de cire dans huile, une huile gélifiée, un gel aqueux, pigmenté ou non.

La composition de l'invention peut comprendre, en outre, d'autres ingrédients usuellement utilisés dans les domaines concernés, choisis parmi les solvants, les épaississants ou gélifiants de phase aqueuse ou de phase huileuse, les matières colorantes solubles dans le milieu de la composition, les particules solides du type charges ou pigments, les antioxydants, les conservateurs, les parfums, les électrolytes, les neutralisants, les agents bloqueurs d'U.V. comme les filtres solaires, les polymères filmogènes, les actifs cosmétiques et pharmaceutiques à action bénéfique pour la peau ou les phanères et notamment les fibres kératiniques (comme les vitamines), leurs mélanges. Ces additifs peuvent être présents dans la composition selon les quantités généralement utilisées dans le domaine cosmétique et dermatologique et notamment à raison de 0,01 à 50 % du poids total de la composition et mieux de 0,1 à 20 % et par exemple de 0,1 à 10 %. Ces adjuvants, selon leur nature, peuvent être introduits dans la phase grasse, dans la phase aqueuse et/ou dans les vésicules lipidiques et notamment des liposomes.

Bien entendu l'homme du métier veillera à choisir les éventuels additifs complémentaires et/ou leur quantité de telle manière que les propriétés avantageuses de la composition selon l'invention, notamment à savoir l'augmentation de la densité des fibres kératiniques, ne soient pas ou substantiellement pas, altérées par l'adjonction envisagée.

Comme solvants utilisables dans l'invention, on peut citer les alcools inférieurs de C₂ à C₈ comme l'éthanol, l'isopropanol, le propylène glycol et certaines huiles cosmétiques légères comme les alcanes en C₆ à C₁₆.

Comme huiles utilisables dans l'invention, on peut citer les huiles d'origine minérale (huile de vaseline, isoparaffine hydrogénée), les huiles d'origine végétale (fraction liquide du beurre de karité, huile de tournesol, d'abricot, de soja, alcool ou acide gras), les huiles d'origine animale (perhydrosqualène), les huiles de synthèse (esters d'acide gras, huile de Purcellin), les huiles siliconées (polydiméthylsiloxanes linéaires ou cycliques, phényltriméthicones) et les huiles fluorées (perfluoropolyéthers). Comme cires, on peut citer les cires siliconées, les cires d'abeille, de candellila, de riz, de carnauba ou de paraffine ou de polyéthylène.

Comme émulsionnants utilisables dans l'invention, on peut citer par exemple le stéarate ou laurate de glycérol, les stéarates ou oléates de sorbitol, les alkyl diméthiconecopolyol (avec alkyle ≥ 8) et leurs mélanges pour une émulsion E/H. On peut aussi utiliser le monostéarate ou monolaurate de polyéthylène glycol, le stéarate ou oléate de sorbitol polyoxyéthyléné, les diméthiconecopolyols et leurs mélanges pour une émulsion H/E.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les Bentones, les sels métalliques d'acides gras comme les stéarates d'aluminium, la silice traitée hydrophobe, l'éthylcellulose, leurs mélanges.

La composition peut contenir un autre actif additionnel que les composés de formule (I) qui peut être hydrophile et choisi parmi les protéines ou les hydrolysats de protéine, les acides aminés, les polyols, l'urée, l'allantoïne, les sucres et les dérivés de sucre, les vitamines hydrosolubles, les extraits végétaux (ceux d'Iridacées ou de soja) et les hydroxy-acides (acide de fruit, acide salicylique) ; ou lipophile et choisi parmi le rétinol (vitamine A) et ses dérivés notamment ester (palmitate de rétinol), le tocophérol (vitamine E) et ses dérivés notamment ester (acétate ou palmitate de tocophérol), les acides gras essentiels, les céramides, les huiles essentielles, les dérivés de l'acide salicylique comme le n-octanoyl-5 salicylique, les esters des hydroxy acides, les phospholipides comme la lécithine, leurs mélanges.

Selon un mode particulier de réalisation de l'invention, on peut associer au composé de formule (I) ou de l'un de ses sels, au moins un composé additionnel favorisant la repousse et/ou limitant la chute des cheveux. Ces composés additionnels sont notamment choisis parmi les inhibiteurs de lipoxygénase tels que décrits dans EP 0 648 488, les inhibiteurs de bradykinine décrits notamment dans EP 0 845 700, les prostaglandines et leurs dérivés notamment ceux décrits dans WO 98/33497, WO 95/11003, JP 97-100091, JP 96-134242, les agonistes ou antagonistes des récepteurs des prostaglandines, les analogues non prostanoïques de prostaglandines tels que décrits dans EP 1 175 891 et EP 1 175 890, WO 01/74307, WO 01/74313, WO 01/74314, WO 01/74315 ou WO 01/72268, leurs mélanges.

Comme autres composés additionnels favorisant la pousse du cheveu pouvant être présents dans la composition selon l'invention on peut citer les vasodilatateurs, les antiandrogènes, les cyclosporines et leurs analogues, les antimicrobiens et antifongiques, les anti-inflammatoires, les rétinoïdes, seuls ou en mélange.

Les vasodilatateurs utilisables sont notamment les agonistes des canaux potassium incluant le minoxidil ainsi que les composés décrits dans les brevets US 3,382,247, 5,756,092, 5,772,990, 5,760,043, 5,466,694, 5,438,058, 4,973,474, la cromakalim, le nicorandil et le diaxozide, seuls ou en association.

Les antiandrogènes utilisables incluent notamment les inhibiteurs stéroïdiens ou non stéroïdiens de 5α-réductase, comme le finastéride et les composés décrits dans US 5,516,779, l'acétate de cyprostérone, l'acide azélaïque, ses sels et ses dérivés et les composés décrits dans US 5,480,913, le flutamide et les composés décrits dans les brevets US 5,411,981, 5,565,467 et 4,910,226.

Les composés antimicrobiens ou antifongiques peuvent être choisis parmi les dérivés du sélénium, le kétoconazole, l'octopirox, le triclocarban, le triclosan, le pyrithione zinc, l'itraconazole, l'acide asiatique, l'hinokitiol, la mipirocine, les tétracyclines, notamment l'érythromycine et les composés décrits dans EP 0 680 745, le chlorhydrate de clinycine, le peroxyde de benzoyle ou de benzyle et la minocycline.

Les anti-inflammatoires peuvent être choisis parmi les anti-inflammatoires stéroïdiens comme les glucocorticoïdes, les corticostéroïdes (par exemple :
l'hydrocortisone) et les anti-inflammatoires non stéroïdiens comme l'acide glycyrrhétinique et l'α-bisabolol, la benzydamine, l'acide salicylique et les composés décrits dans EP 0 770 399, WO 94/06434 et FR 2 268 523.

Les rétinoïdes peuvent être choisis parmi l'isotrétinoïne, l'acitrétine et le tazarotène.

Comme autres composés additionnels actifs pour favoriser la pousse et/ou limiter la chute des cheveux utilisables en association avec le composé de formule (I), salifié ou non, on peut citer l'aminexil, le 6-0-[(9Z,12Z)-octadéca-9,12-diènoyl]hexapyranose, le chlorure de benzalkonium, le chlorure de benzéthonium, le phénol, l'oestradiol, le maléate de chlorphéniramine, les dérivés de chlorophylline, le cholestérol, la cystéine, la méthionine, le menthol, l'huile de menthe poivrée, le panthoténate de calcium, le panthénol, le résorcinol, les activateurs de la protéine kinase C, les inhibiteurs de la glycosidase, les inhibiteurs de glycosaminoglycanase, les esters d'acide pyroglutamique, les acides hexosaccharidiques ou acyl-hexosaccharique, les éthylènes aryl substitués, les amino-acides N-acylés, les flavonoïdes, les dérivés et analogues d'ascomycine, les antagonistes d'histamine, les saponines, les inhibiteurs de protéoglycanase, les agonistes et antagonistes d'estrogènes, les pseudotèrines, les cytokines et les promoteurs de facteurs de croissance, les inhibiteurs d'IL-1 ou d'IL-6, les promoteurs d'IL-10, les inhibiteurs de TNF, les hydroxy-acides, les benzophénones et l'hydantoïne, l'acide rétinoïque ; les vitamines comme la vitamine D, les analogues de la vitamine B12 et le panthoténol ; les triterpènes comme l'acide ursolique et les composés décrits dans US 5,529,769, US 5,468,888, US 5,631,282 ; les agents antiprurigineux comme la thénaldine, la triméprazine ou la cyproheptadine ; les antiparasitaires, en particulier le métronidazole, le crotamiton ou les pyréthrinoïdes ; les antifongiques, en particulier l'octopirox et les composés appartenant à la classe des imidazoles tels que l'éconazole, le kétoconazole ou le miconazole ou leurs sels, les esters d'acide nicotinique, dont notamment le nicotinate de tocophérol, le nicotinate de benzyle et les nicotinates d'alkyles en C₁-C₆ comme les nicotinates de méthyle ou d'hexyle ; les agents antagonistes de calcium, comme la cinnarizine, le diltiazem, la nimodipine, vérapamil, l'alvérine et la nifédipine ; les hormones telles que l'estriol ou ses analogues, la thyroxine et ses sels, la progestérone ; les agents antiandrogènes tels que l'oxendolone, la spironolactone, le diéthylstilbestrol et la flutamide ; les agonistes du récepteur FP (récepteur aux prostaglandines du type F) tels que le latanoprost, le bimatoprost, le travoprost, l'unoprostone ; les inhibiteurs de 15-hydroxyprostaglandine désydrogénase ; leurs mélanges.

On peut également envisager que la composition comprenant au moins le composé de formule (I), salifié ou non soit sous forme liposomée, telle que notamment décrite dans le document WO 94/22468. Ainsi, le composé encapsulé dans les liposomes peut être délivré sélectivement au niveau du follicule pileux.

### Procédé cosmétique et utilisation

La composition selon l'invention peut être appliquée sur les zones alopéciques du cuir chevelu et des cheveux d'un individu, et éventuellement laissée en contact plusieurs heures et éventuellement rincée.

On peut, par exemple, appliquer la composition contenant une quantité efficace d'un composé de formule (I), salifié ou non, le soir, garder celle-ci au contact toute la nuit et éventuellement effectuer un shampooing le matin. Ces applications peuvent être renouvelées quotidiennement pendant un ou plusieurs mois suivant les individus.

Avantageusement, dans le procédé selon l'invention, on applique sur les zones à traiter du cuir chevelu entre 5 et 500 µl d'une solution ou composition telle que définie précédemment, comprenant de 0,001 % à 5 % de composé de formule (I).

Les exemples qui suivent illustrent l'invention sans toutefois en limiter la portée.

### Exemple 1 :

Le composé 1 a été synthétisé selon la méthode décrite dans DE 924 085.

La molécule testée (1) a été étudiée versus son analogue commercial (1') sans la fonction nitrate, comme référence négative :

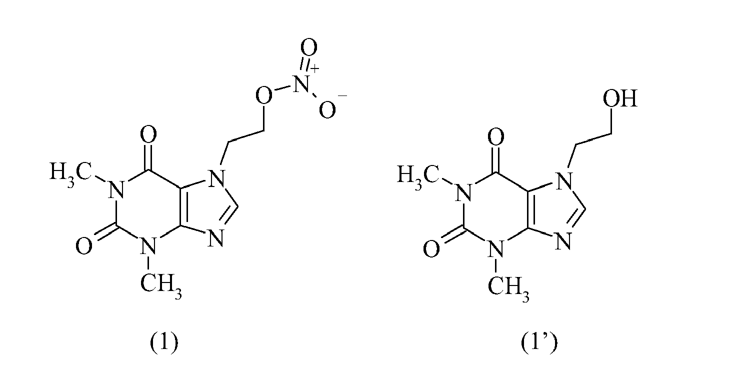

### Exemple 1.1 : Stabilité chimique en solution du composé (1)

Les études de stabilité ont été effectuées sur une durée de 2 mois à 45 °C en solution dans le milieu éthanol / isopropanol / eau : 40/10/50 à la concentration de 0,2 %.

L'étude est suivie en HPLC.

| | |
|---|---|
| *Perte en % de produit à 4 °C* | *Non significative* |
| *Perte en % de produit à 45 °C* | *Non significative* |

Il a bien été confirmé par cette étude que le composé (1) est stable en solution.

### Exemple 1.2. : Activité donneur de NO du composé (1) par rapport au composé (1')

L'activité des composés (1) et (1') sur la NO-synthase inductible a été évaluée dans le test décrit par Heck et col. (J.B.C., Vol. 267, N° 30, 21277-21280, 25 octobre 1992), test de « Modulation de l'induction de la NOS₂ (Nitric Oxide Synthase inductible) sur kératinocytes humains normaux». Ce test a pour objectif d'évaluer la concentration en nitrates et nitrites, *in fine*, après stimulation de la NO-synthase 2 ou application d'un donneur de NO ; il est réalisé par comparaison avec la stimulation de la NO-synthase inductible par des cytokines. La concentration en nitrates et en nitrites, in fine, correspond à la concentration de NO libéré.

### MATERIEL ET METHODE

Le test est effectué sur une culture de kératinocytes humains normaux, issus de prélèvements. L'induction de la NO-synthase inductible (NOS₂) a été provoquée par l'addition d'une combinaison de plusieurs cytokines au milieu de culture. Les produits testés ont été appliqués à trois concentrations variant de 10⁻⁵ à 10⁻³M.

### Les contrôles suivants ont été introduits dans le test :

A : contrôle positif (induction de l'enzyme sur cellules stimulées) :
mélanges d'interféron-γ et d'interleukine 1-β correspondant à 100 % de stimulation.

B : contrôle basal des cellules non stimulées (sans cytokines), correspondant à 0 % de stimulation.

### MESURES ET RESULTATS

Pour déterminer l'activité des produits à tester, on mesure la quantité de produits de réaction stables du NO (nitrites et nitrates) à l'aide du kit « Nitric ColorimetricAassay » vendu par la société Boehringer sous la référence 1756.28.

Les composés ont été tests aux concentrations de 10⁻³M/10⁻⁴M et 10⁻⁵M dans l'éthanol (moyenne n=3).

| **Produit testé** | **Concentration** | **% stimulation sur cellules stimulées** | **% stimulation sur cellules non stimulées** |
|---|---|---|---|
| A | | 100 | / |
| B | | 0 | 0 |
| **Composé (1)** | 10⁻⁵M | 101,57 % | 12,17 % |
| **Composé (1)** | 10⁻⁴M | 150,10 % | 70,13 % |
| **Composé (1)** | 10⁻³M | 432,13 % | 374,07 % |
| **Composé (1')** | 10⁻⁵M | 115,13 %=SE | SE |
| **Composé (1')** | 10⁻⁴M | 99,83 %=SE | SE |
| **Composé (1')** | 10⁻³M | 100,53 %=SE | SE |

| | | | |
|---|---|---|---|
| * SE signifie : sans effet (modulation inférieure à 10 %) | | | |

Du tableau ci-dessus, on conclut que le composé (1) présente un effet donneur de NO, sur cellules stimulées ou non, à l'inverse du composé (1').

### Exemple 2 : Composition anti-chute de cheveux/lotion capillaire

- Composé (1) 1,00 %
- Propylène glycol 30,00 %
- Alcool éthylique 40,00 %
- Eau qsp 100,00 %

On applique cette lotion sur le cuir chevelu, une à deux fois par jour, à raison d'1 ml par application, en massant légèrement le cuir chevelu pour faire pénétrer l'actif. La chevelure est ensuite séchée à l'air libre. Cette lotion permet de diminuer la chute des cheveux et de favoriser leur repousse.

## Revendications

1. Utilisation cosmétique d'au moins un dérivé N-oxyde de la formule (1) 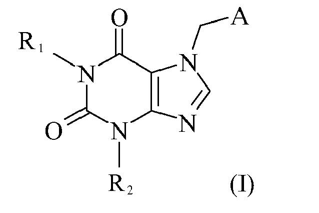 où A peut prendre l'une des deux formules (II) ou (III) suivantes : 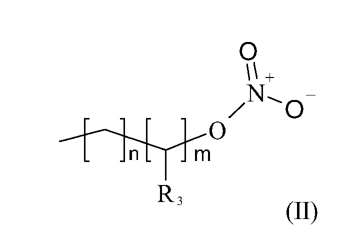 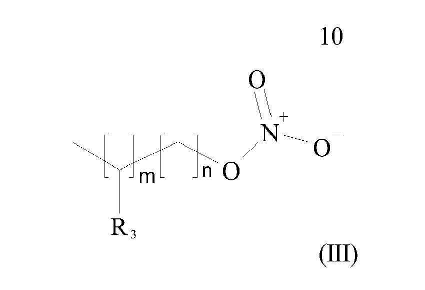 et dans laquelle :
- n représente un entier allant de 0 à 5,
- m représente un entier allant de 0 à 1, à condition que m et n ne soient pas égaux à 0 simultanément,
- R₁ et R₂ représentent indépendamment un radical (C₁-C₅)alkyle, linéaire, ramifié ou cyclique, saturé ou insaturé et,
- R₃ représente un groupe (C₁-C₄)alkyle, un groupe hydroxy, un groupe (C₁-C₄)alkoxy ou un groupe nitrate,
comme agent précurseur de NO.

2. Utilisation selon la revendication 1, **caractérisée en ce que** le composé de formule (I) est destiné à induire et/ou stimuler la croissance des fibres kératiniques et/ou des cils et/ou freiner leur chute.

3. Utilisation d'un composé de formule (I), tel que défini dans la revendication 1 ou 2, dans une composition cosmétique de soin capillaire humain, pour réduire la chute des cheveux et/ou augmenter leur densité et/ou traiter l'alopécie androgénique.

4. Utilisation selon l'une quelconque des revendications 1 à 3, **caractérisée en ce que** R₁ et R₂ représentant indépendamment un groupe (C₁-C₅) alkyle non substitué.

5. Utilisation selon l'une quelconque des revendications 1 à 4, **caractérisée en ce que** R₁ et R₂ sont chacun un groupe méthyle.

6. Utilisation selon la revendication 5, **caractérisée en ce que** m est égal à 0, n est un entier allant de 1 à 3, et plus particulièrement est égal à 1.

7. Utilisation cosmétique d'au moins un composé de formule (I) ou de l'un de ses sels selon l'une quelconque des revendications 1 et 4 à 6, dans une composition cosmétique de soin capillaire d'être humain, comme agent pour traiter l'alopécie d'origine naturelle et en particulier l'alopécie androgénique ou andro-chrono-génétique.

8. Utilisation d'au moins un composé de formule (I) ou de l'un de ses sels selon l'une quelconque des revendications 1 et 4 à 6, pour la préparation d'une composition capillaire d'être humain, destinée à traiter l'alopécie d'origine naturelle et en particulier androgénique ou andro-chrono-génétique.

9. Utilisation cosmétique d'au moins un dérivé de formule (I) ou de l'un de ses sels selon l'une quelconque des revendications 1 et 4 à 6, dans une composition cosmétique de soin et/ou de maquillage des cils d'être humain ou pour la préparation d'une composition de soin et/ou de traitement des cils d'être humain, pour ou destinée à induire et/ou stimuler la croissance des cils et/ou augmenter leur densité.

10. Utilisation d'au moins un composé de formule (I) ou l'un de ses sels selon l'une quelconque des revendications 1 et 4 à 6, pour la préparation d'une composition de soin et/ou de traitement des fibres kératiniques d'êtres humains, destinée à induire et/ou stimuler la croissance des fibres kératiniques et/ou freiner leur chute et/ou augmenter leur densité.

11. Utilisation d'au moins un dérivé N-oxyde de la théophylline de formule (I) ou de l'un de ses sels tels que définis selon l'une quelconque des revendications 1 et 4 à 6, pour la fabrication d'une composition destinée à traiter les désordres liés à une réduction de la microcirculation ou de la microvascularisation cutanée et notamment des follicules pileux chez l'être humain.

12. Utilisation cosmétique d'au moins un dérivé de formule (I) ou l'un de ses sels selon l'une quelconque des revendications 1 et 4 à 6, dans une composition cosmétique anti-âge, anti-ride, amincissante ou conférant à la peau un éclat du teint ou une luminosité du teint ou aux lèvres de la couleur.

13. Utilisation d'au moins un composé de formule (I) ou l'un de ses sels selon l'une quelconque des revendications 1 et 4 à 6, pour la préparation d'une composition physiologiquement acceptable, destinée à traiter les signes du vieillissement intrinsèques et/ou extrinsèques, les surcharges pondérales, les ulcères, le syndrome de Raynaud, le psoriasis et/ou améliorer la cicatrisation cutanée.

14. Utilisation d'au moins un composé de formule (I) ou l'un de ses sels selon l'une quelconque des revendications 1 et 4 à 6, pour la fabrication d'une composition physiologiquement acceptable, destinée à traiter les mycoses cutanées, les désordres inflammatoires, les réactions d'hypersensibilité de contact, les manifestations allergiques, l'eczéma, les prurits, les peaux sensibles et/ou l'hypermélanose.

15. Composé de formule (I) ou l'un de ses sels tel que défini selon l'une quelconque des revendications 1 et 4 à 6, à l'exclusion du composé non salifié pour lequel A prend la formule (III), n est égal à 1, m est égal à 0 et R₁ et R₂ représentent chacun un groupe méthyle ; du composé non salifié pour lequel A prend la formule (III), n et m sont égaux à 1, R₁ et R₂ représentent chacun un groupe méthyle et R₃ représente un groupe hydroxyle ; du composé non salifié pour lequel A prend la formule (III), n et m sont égaux à 1, R₁ et R₂ représentent chacun un groupe méthyle et R₃ représente un groupe nitrate ; du composé non salifié pour lequel A prend la formule (III), n est égal à 2, m est égal à 0 et R₁ et R₂ représentent chacun un groupe méthyle et du composé non salifié pour lequel A prend la formule (III), n est égal à 0, m est égal à 1, R₁ et R₂ représentent chacun un groupe méthyle et R₃ représente un groupe méthyle.

16. Composition cosmétique comprenant au moins un composé de formule (I) tel que défini selon l'une quelconque des revendications 1 et 4 à 6.

17. Composition selon la revendication précédente, **caractérisée en ce que** la composition est une composition à application topique.

18. Composition dermatologique contenant dans un milieu physiologiquement acceptable une quantité efficace d'au moins un composé de formule (I) ou l'un de ses sels selon l'une quelconque des revendications 1 et 4 à 6, à l'exclusion du composé non salifié pour lequel A prend la formule (III), n est égal à 1, m est égal à 0 et R₁ et R₂ représentent chacun un groupe méthyle ; du composé non salifié pour lequel A prend la formule (III), n et m sont égaux à 1, R₁ et R₂ représentent chacun un groupe méthyle et R₃ représente un groupe hydroxyle ; du composé non salifié pour lequel A prend la formule (III), n et m sont égaux à 1, R₁ et R₂ représentent chacun un groupe méthyle et R₃ représente un groupe nitrate ; du composé non salifié pour lequel A prend la formule (III), n est égal à 2, m est égal à 0 et R₁ et R₂ représentent chacun un groupe méthyle et du composé non salifié pour lequel A prend la formule (III), n est égal à 0, m est égal à 1, R₁ et R₂ représentent chacun un groupe méthyle et R₃ représente un groupe méthyle.

19. Composition dermatologique topique contenant dans un milieu physiologiquement acceptable une quantité efficace d'au moins un composé de formule (I) ou l'un de ses sels selon l'une quelconque des revendications 1 et 4 à 6.

20. Composition selon l'une quelconque des revendications 15 à 19, **caractérisée en ce que** le ou les composé(s) de formule (I) est ou sont présent(s) dans une teneur allant de 10⁻³ % à 5 % du poids total de la composition et préférentiellement en une quantité représentant de 10⁻² % à 2 % du poids total de la composition, et de façon encore plus préférée de 0,1 % à 2 %.

21. Procédé de traitement cosmétique des fibres kératiniques humaines et/ou de la peau y compris le cuir chevelu, destiné notamment à stimuler la croissance des fibres kératiniques humaines telles que les cheveux et les cils d'êtres humains et/ou freiner leur chute, **caractérisé par le fait qu'**il consiste à appliquer sur les fibres kératiniques humaines et/ou la peau, une composition cosmétique comprenant une quantité efficace d'au moins un composé de formule (I) telle que définie selon l'une quelconque des revendications 1 et 4 à 6 ou d'un de ses sels, à laisser celle-ci en contact avec les fibres kératiniques et/ou la peau, et éventuellement à rincer les fibres kératiniques et/ou la peau.

22. Procédé de soin cosmétique des cheveux et/ou du cuir chevelu humains, en vue d'améliorer leur état et/ou leur aspect, **caractérisé en ce qu'**il consiste à appliquer sur les cheveux et/ou le cuir chevelu, une composition cosmétique comprenant au moins un dérivé de formule (I) telle que définie selon l'une quelconque des revendications 1 et 4 à 6 ou l'un de ses sels, à laisser celle-ci au contact des cheveux et/ou du cuir chevelu, et éventuellement à rincer les cheveux et/ou le cuir chevelu.

23. Procédé de soin cosmétique et/ou de maquillage des cils humains, en vue d'améliorer leur état et/ou leur aspect, **caractérisé en ce qu'**il consiste à appliquer une composition de mascara comprenant au moins un composé de formule (I) telle que définie selon l'une quelconque des revendications 1 et 4 à 6 ou l'une de ses sels et à laisser celle-ci au contact des cils.
